# EUROPEAN PATENT APPLICATION

(11) **EP 2 468 345 A1**
(43) Date of publication of application: **27.06.2012**
(21) Application number: 10196956.6
(22) Date of filing: 23.12.2010
(51) Int. Cl.: A61M 11/06, A61M 16/18

(54) **Improved injection vaporizer and method of vaporization control**

(71) Applicant: Maquet Critical Care AB, 17106 Solna (SE)
(72) Inventor: Larsson, Åke, 17552, Järfälla (SE); Troili, Carl, 18256, Danderyd (SE)
(74) Representative: KIPA AB

(57) **Abstract**

An injection device and method of controlling the injection is disclosed for vaporizing a liquid substance in a breathing apparatus. The device comprises a vaporizer chamber that has an interior surface with at least a first surface portion. Further, an injecting unit is provided for generating a spray of said liquid substance into said vaporizer chamber towards said first surface portion. A dispersal unit provides for contactless acting on at least a portion of said spray of said liquid in said vaporizer chamber relative said first receiving surface portion when injected into said vaporizer chamber. Thus vaporization is made in an efficient, controllable way.

## Description

### Field of the Invention

This invention pertains in general to the field of breathing apparatuses. More particularly the invention relates to injection vaporizers in such breathing apparatuses.

### Background of the Invention

In published European Patent application number EP1402916A1 of the same applicant as the present application, an injection vaporizer is disclosed that has a reservoir for liquid to be vaporized and a delivery conduit connected to the reservoir with an outlet for injecting liquid to be vaporized into a gas space, formed by a flow-through chamber. A pumping system is operable to transfer liquid from the reservoir to the outlet.

A modern injection vaporizer, such as disclosed in EP1402916A1, has high demands with regard to quickness of the vaporizing process. In such an injection vaporizer, a small amount of liquid anesthetic agent, such as halogenated anesthetic agents as for instance Halothane, Sevoflurane, Isoflurane, Enflurane, or Desflurane, is injected or sprayed into a vaporizer chamber. This is made by means of a technique atomizing the liquid into small droplets. The mean flow of the liquid is controlled by a variation of the dosing pulse length, as well as the pulse frequency. A typical dosing pulse is approximately between 2 to 10 msec.

The vaporization rate of the droplets in turn depends on the amount of heat energy available for providing the transformation from the liquid phase to the gaseous phase of the anesthetic agent. It depends further on the removal rate of the gaseous medium from the surface of the liquid droplet to keep the vaporizing process ongoing until the droplet is completely gasified.

Known injection vaporizers are based on a temperature regulation of the temperature of the vaporization chamber that only control the mean temperature therein in a slow process. Temperature regulation can thus not follow the quick injection process and related vaporization events following the aforementioned extremely short injection pulses.

The power that would be needed to provide the heat energy necessary to instantly gasify injected liquid is in the range of approximately 60 to 80 Watts. There are a number of reasons to reduce such energy consumption. Medical devices are for instance required to have battery backup units for safety reasons. Reduced energy consumption of a breathing apparatus having an injection vaporizer does thus imply downsized energy sources for the apparatus. This means the size and cost of backup units, main power supplies, can be reduced. Reduced energy consumption is also an environmental issue, i.e. the life cycle burden on the environment is improved by reducing the necessary energy consumption. of the breathing apparatus.

Moreover, it might be an issue to provide the necessary energy to the site of evaporation, often inside a vaporization chamber that is cooled by incoming gas to be delivered to a patient. It would be advantageous to be able to controllably convey anesthetic agent liquid to various different evaporation sites in the injection vaporizer.

The flow of gas leaving the vaporizer to the patient should have as much of the liquid gasified therein as possible. It should be avoided to have remaining liquid particles in this gas flow. For instance droplets leaving the injection vaporizer to the patient should avoided. The number of droplets should be minimized or be as low as possible, preferably none at all. In this manner, it would be avoided that any anesthetic agent leaving the injection vaporizer in the gas flow would uncontrollably be condensing in the gas channels of a breathing apparatus or patient tubing.

Moreover, temperature regulation is slow as feedback is thermistor based. The thermistor has a time constant of several seconds and temperature regulation thus in the range of Hz. This allows only for a correspondingly slow temperature regulation process of the mean temperature in the vaporization chamber.

Vaporization in an injector vaporizer thus takes place in two stages. A minor portion of the liquid gasifies during the flight from the injector towards an interior wall of a vaporizer chamber in the injection vaporizer unit. The major portion of the injected liquid is gasified in a secondary stage from the wall of the vaporizer chamber.

As injection nozzles that are used today have a narrow spray lobe with a resulting fixed spreading angle. Therefore, the injected liquid collects on a small surface area of the interior wall of the vaporizer chamber, to which surface the spray lobe is directed. Hence, due to a low thermal capacity of the volume available for the vaporization process temperature drops quickly in the vaporization chamber following the injection pulse. This temperature drop may lead to such low temperatures that the vaporization of the liquid injected anesthetic agent ceases. This would be undesired in clinical operation.

Thus, there is a need for an improved injection vaporizer. The improved injection vaporizer should advantageously provide effective vaporization.

### Summary of the Invention

Accordingly, embodiments of the present invention preferably seek to mitigate, alleviate or eliminate one or more deficiencies, disadvantages or issues in the art, such as the above-identified, singly or in any combination by providing an injection vaporizer, and a method of controlling an injection vaporizer, according to the appended patent claims.

Embodiments provide for controllably distributing anesthetic agent liquid to desired sites of evaporation in an anesthetic injection vaporizer. The spray from an injection nozzle unit in an injection vaporizer is controllably delivered to desired areas in the vaporizer chamber. This facilitates the vaporization process and makes it more effective than in conventional injection vaporizers.

A flow of gas leaving the vaporizer to the patient has effectively the liquid gasified therein. It is thus avoided to have remaining liquid particles in this gas flow. It is avoided that droplets leave the injection vaporizer to the patient. It is effectively avoided that leaves the injection vaporizer in the gas flow. Thus, it is avoided that anesthetic agent liquid uncontrollably condenses in the gas channels of a breathing apparatus or patient tubing thereof.

This improved efficiency is provided by anesthetic agent liquid being controllably delivered to desired surface areas of the vaporizer for evaporation from these surfaces. The surfaces are for instance set to controlled electrical potentials to controllably direct the droplets in the vaporizer. Vaporization is thus provided in a controlled and effective manner.

In some embodiments, the liquid substance to be injected is electrically charged to a certain first potential. At least a portion of the vaporizer chamber interior wall is electrically charged to a different potential. The potential difference supports the distribution of the liquid substance in the vaporization chamber. In this manner the liquid is selectively distributable to selected areas of the interior wall of the vaporizer chamber. Electrical attraction and repelling forces are provided to control the distribution.

For instance, the surface area on which injected liquid substance droplets normally, when their trajectory is not influenced upon injection, land may be provided with an opposite polarity as the injected liquid. This provides for a repelling force providing for spreading the liquid over a larger surface. Alternatively, or in addition, different portions of the vaporizer chamber interior wall may selectively be provided with different electrical potential. Alternatively, or in addition, different electrical potentials may be provided during different time phases of the injection and/or vaporization of the anesthetic liquid.

In some embodiments, the fact that certain anesthetic agent substances have a moment of dipole is taken advantage of. Electrical attraction or repelling forces are provided by only controlling a potential of receiving surfaces. The liquid itself needs not, and is not, provided with a specific potential. This allows for a controlled deflection of a spray of anesthetic agent liquid to improve vaporization. Thus, a potential of certain components of the vaporizer needs not to be controlled. It can be set to zero or ground level. These parts may include an injection unit, such as a nozzle, a liquid flow channel, and/or a container for holding or conveying the anesthetic liquid.

Thus, the collection of an injected liquid is effectively avoided. The effective surface area on which the yet not gasified liquid substance is collected is considerably increased. Efficiency of the injection vaporizer is increased. The vaporization process is efficiently controllable.

In addition, the injected spray may be directed onto an enlarged evaporation surface to further increase efficiency of the vaporization of the anesthetic agent liquid. The evaporation surface may comprise an enlarged surface area. The enlarged surface area may comprise one or more conical portion(s) of the interior surface of the gas channel into which a spray is generated. A conical evaporation surface may in addition be enlarged by secondary enlarged surface areas. The enlarged surface area of evaporation surface may for this purpose be corrugated or porous. The enlarged surface area of evaporation surface allows for an increased spreading of the liquid to a larger surface area and thus further improved vaporization efficiency.

A porous surface may be made of a sintered porous unit, such as commercially available under the name "sica fil". The porous surface may alternatively be of aluminum foam. The aluminum foam is produced by a stabilized aluminum foaming process where air is bubbled through liquid aluminum which in turn is solidified into the desired shape. The porous surface is preferably of electrically conductive material. Further, the porous surface is preferably of a thermally well conductive material to effectively heat the evaporation surface. A heater element may be in thermal contact with the evaporation surface.

According to a first aspect of the invention, an injection vaporizer is provided. The injection vaporizer is an injection device for vaporizing a liquid substance in a breathing apparatus. The device comprises a vaporizer chamber that has an interior surface with at least a first surface portion, an injecting unit for generating a spray of the liquid substance into the vaporizer chamber towards the first surface portion, and a dispersal unit for contactless acting on at least a portion of the spray of the liquid in the vaporizer chamber relative the first receiving surface portion when injected into the vaporizer chamber.

According to a second aspect of the invention, a method of controlling an injection vaporizer is provided. The method is a method of internally controlling the injection device for improving vaporization of a liquid substance. The method comprises injecting a spray of the liquid substance into a vaporizer chamber towards a first surface portion of an interior surface of the vaporizer chamber, and contactless acting on at least a portion of the spray of the liquid in the vaporizer chamber for dispersing at least the portion of the spray relative the first receiving surface portion when injected into the vaporizer chamber.

According to a further aspect of the invention, a computer-readable medium having embodied thereon a computer program for processing by a computer is provided. The computer program is preferably embodied on a computer-readable medium. The computer program may enable carrying out of a method according to the second aspect of the invention. The computer program comprises a code segment for controlling a dispersal unit for increasing dispersal of the spray based on a temperature drop in the vaporizer chamber upon injection of the spray, when injecting a spray of the liquid substance into a vaporizer chamber towards a first surface portion of an interior surface of the vaporizer chamber, and whereby dispersal is made by the dispersal unit by contactless acting on at least a portion of the spray of the liquid in the vaporizer chamber for dispersing at least the portion of the spray relative a first receiving surface portion when injected into the vaporizer chamber.

Further embodiments of the invention are defined in the dependent claims, wherein features for the second and subsequent aspects of the invention are as for the first aspect mutatis mutandis.

The term "spray" as used in the present context means
a liquid broken up into minute droplets and blown, ejected into, or falling through a gaseous medium; or a jet of fine particles of liquid, discharged from an injection unit, such as a nozzle or an atomizer or other device into a chamber and/or for application to a surface. The spray may be provided as an aerosol.

The term "disperse" as used in the present context means to separate and move in different selected directions in particular scatter; or to turn aside from a path or course; or deflect; or turn from a true course or straight line.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Fig. 1 is a schematic view illustrating an embodiment of an injection vaporizer;
Fig. 2 is a schematic view illustrating another embodiment of an injection vaporizer
Fig. 3 is a schematic illustration of towards each other isolated surface portions;
Fig. 4 is a schematic illustration of a breathing apparatus comprising an injection device;
Fig. 5 is a flow chart of a method; and
Fig. 6 is a schematic illustration of a computer program.

### Description of embodiments

Specific embodiments of the invention now will be described with reference to the accompanying drawings. This invention may, however, be embodied in many different forms and should not be construed as limited to the embodiments set forth herein; rather, these embodiments are provided so that this disclosure will be thorough and complete, and will fully convey the scope of the invention to those skilled in the art. The terminology used in the detailed description of the embodiments illustrated in the accompanying drawings is not intended to be limiting of the invention. In the drawings, like numbers refer to like elements.

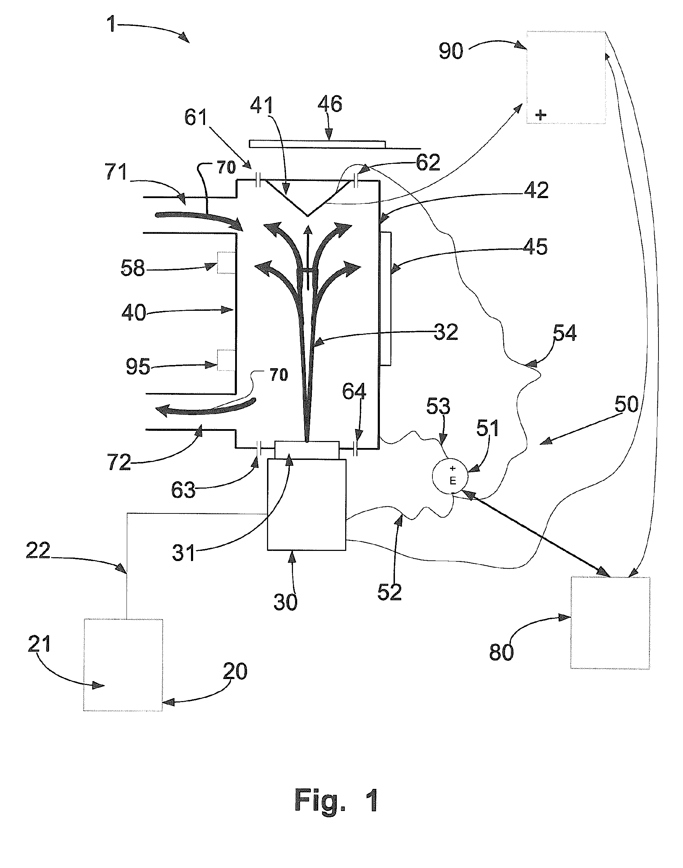

In an embodiment of the invention according to Fig. 1, an injection vaporizer is shown. The injection vaporizer is in the illustrated embodiment an injection device 1 for vaporizing a liquid substance 21 in a breathing apparatus 200. A vaporizer chamber 40 has an interior surface with at least a first surface portion 41. An injecting unit 30 for generating a spray 32 of the liquid substance into the vaporizer chamber 40 towards the first surface portion 41. The injection unit 30 may comprise an injector, which for instance is piezo based; a pump, such as a micropump; or other units suitable to convey a desired of amount of liquid to a vaporization chamber from a vessel, preferably intermittently. A dispersal unit 50 is provided for contactless acting on at least a portion of the spray 32 of the liquid in the vaporizer chamber relative the first receiving surface portion when injected into the vaporizer chamber.

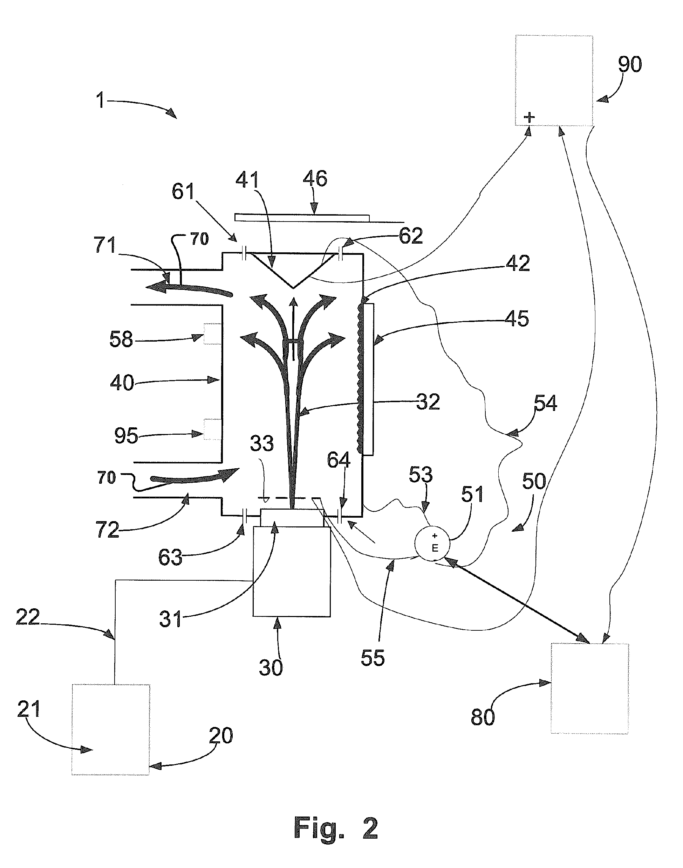

The spray 32 is preferably an intermittent or pulsed spray. It is preferably provided only during phases when a gas flow exists between a first gas port 71 of the vaporizer chamber 40 and further to a second gas port 72. The first gas port 71 may in embodiments be an inlet (Fig. 1) or an outlet (Fig. 2). The direction of the gas flow 70 may be in the same direction, opposite to, or at an arbitrary angle in relation to the injection direction of the spray 32. The gas flowing through and out of the chamber 40 is thus enriched with the gasified substance. In other embodiments (not shown) of the vaporizer chamber, the gas may pass in other arrangements in and out of the vaporizer chamber. The chamber may for instance be semi-open where an inflow and outflow passes transversely through the chamber. The chamber may also have one gas port only, where the chamber is filled with unsaturated gas before injecting the liquid. After an evaporation phase, the saturated gas is then released through the same gas port.

The spray 32 is altered in its flow direction by the dispersal unit 50. It is provided for controllably distributing the droplets of the spray in the vaporizer chamber. Thus, a control of a movement, or a trajectory of at least a portion of the droplets of the spray injected into the vaporizer chamber 40 is provided. In this manner, the vaporizing process of the liquid substance is facilitated. The vaporizing efficiency is improved. Thus, a larger amount of liquid substance may be vaporized in comparable times than without the dispersal unit 50.

The spray may thus be directed towards one or more desired surface portions of the interior of the vaporizer chamber 40. At least a portion of the spray may thus be selectively directed towards a receiving target surface, different from the first surface portion 41.

The portion of the liquid spray during its flight from the injector nozzle 31 to the target surface 41 will land on the interior surface of the vaporizer chamber towards which it is directed. The portion of the liquid substance that thus is landed, is advantageously kept in place, e.g. by a potential difference of the evaporation surface relative a potential of the injection unit 30.

As the surface reachable by the spray thus is enlarged, a more effective vaporization is obtained. As the additionally or alternatively reachable surface portions may comprise heater elements 45 in addition to a heater element 46 at the target surface, more heat transfer is provideable to the liquid substance to be vaporized and hence vaporization is improved. The heating unit or heater element is arranged to heat at least the first surface portion and/or the further surface in the vaporizer chamber 40. Thus, more heat energy is provideable for a more effective vaporization.

The spray is selectively distributed, e.g. over a larger area, than without the dispersal unit 50.

The first surface portion is a surface portion in the vaporizer chamber 40 on which droplets of the spray normally will land in operation of the device when not dispersed. The dispersal unit 50 is arranged to direct the portion of the spray towards at least a further surface in the vaporizer chamber, different from the first surface portion. This provides for a larger landing surface, and more effective vaporization. A portion of the spray may still land on the first surface portion, even when the spray is subject to the dispersal unit 50.

The dispersal unit may be active or activated intermittently, synchronized with the injection of the spray 32. This provides for a number of advantages. For instance, energy is saved by the dispersal unit being switched off certain time amounts. Moreover, the intensity or strength of dispersal provided by the dispersal unit may be time variable. It may for instance provide a larger intensity or strength of dispersal during an initial time phase of injection of the spray. It may then be reduced in strength to provide a different dispersal of the spray after the initial time has lapsed. Subsequently, strength may be changed again, e.g. before the pulse of the spray ends. This allows for a further improved distribution of the droplets in the vaporizer chamber 40 and even further effective vaporization of the substance therein.

The intensity or strength of dispersal provided by the dispersal unit may be different for different (or subsequent) pulses. Thus, surface areas to be supplied with liquid for evaporation may be controllably chosen and varied. This may advantageously be made to improve vaporization. The heat energy necessary for evaporation may be provided more effectively as it is distributed between the pulses and different surface portions. This allows for a reduced heating power for each surface portion as more time is available to re-heat the surface after evaporation of the liquid has used the available heat energy for evaporation of the liquid.

As the dispersal unit 50 acts upon the spray without physical contact in embodiments, it is not a mechanical deflector unit or a flow diverter unit based on mechanically acting relative the spray. Uncontrolled condensation of liquid accumulation in the vaporizer chamber is avoided.

In embodiments, the liquid substance is an anesthetic agent. The anesthetic agent may include halogenated anesthetic agents as for instance Halothane, Sevoflurane, Isoflurane, Enflurane, or Desflurane.

The dispersal unit 50 comprises in some embodiments an electric charge unit 51 for providing a first electrical potential to at least a portion of the interior surface. A second electrical potential, different from the first electrical potential, is given to the liquid substance. Thus a directable force is provideable on liquid substance relative the charged surface by means of the potential difference. For instance, when the liquid substance has a lower potential than the surface, it is attracted to the surface. When the liquid substance has a potential of different polarity than the potential of the surface, it is repelled from that surface. In this manner, the liquid spray is controllably directable as desired towards or away from specific surface portions of the interior of the vaporizer chamber 40.

When electrically charged droplets of the spray are injected into the vaporizer chamber, they will land on a droplet landing position on the interior surface of the vaporization chamber 40. The droplet is then discharged at the landing position, which becomes occupied by the droplet until it is vaporized and leaves the landing position for a new droplet to land. During the vaporization time of the droplet, other droplets of the spray are diverted from the landing position to other landing positions different from the landing position of the droplet. This explains the more effective vaporization that is achieved.

For this purpose, for instance the electric charge unit 51 comprises an electrical potential source operatively in electrical contact with at least one surface portion and the liquid substance to provide it with the second electrical potential, different from the first electrical potential given to the surface portion.

The electrical potential source may be electrically connected with an electrically conducting portion at the injection unit 30, such as of a nozzle 31, a flow channel 22, and/or a container 20 for holding or conveying the liquid 21. Thus the liquid substance is provideable with the second electrical potential such that droplets of the liquid injected into the vaporizer chamber are provideable with the second electrical potential.

The container 20 is a reservoir for the liquid to be vaporized, that may be arranged remote from the injection unit 30.

Alternatively, or in addition, the electrical potential source is operatively in electrical contact with the spray 32 upon injection from the injection unit 30 to provide the liquid injected substance with the second potential.

Alternatively, or in addition, the electrical potential source is operatively in electrical contact with an electrically conductive body, such as a net, in the flow channel 22, nozzle 31 or container 20, arranged to be in electrical contact with the liquid prior to being injected into the vaporizer chamber 40.

Alternatively, or in addition, the electrical potential source is operatively in electrical contact with an electrically conductive body 33, such as a net of electrically conductive material, arranged to contact the spray of the liquid substance upon injection from the injection unit before reaching the first surface portion. This is illustrated in Fig. 2. Preferably, the body 33 is arranged close to the nozzle 31 of the injector unit 30.

The injector device 1 comprises a control unit adapted to vary a difference between the first electrical potential and the second electrical potential for providing an attraction or repulsion of the spray in relation to the interior surface in selectable intensity.

The control unit may also be adapted to vary the potential over time to further improve vaporization efficiency, as described above.

The injector device 1 may comprise a measurement unit 90 for measuring a potential difference between the first and second potential and providing a corresponding signal for further processing.

The control unit 80 may be adapted to control a direction of droplets of the spray 32 by a spatial electric charge distribution in the vaporizer chamber. Depending on a spatial arrangement of the surface portions, and a spatial distribution and intensity of potentials thereof, such as based on the measured potential difference signal, the spray may be directed to different heater elements 45, 46. In addition to the potential difference, the mass of the droplets in the spray, determine the trajectory of the spray and thus the spatial distribution thereof.

The measurement unit 90 may be arranged to measure an electric charge received over time at the interior surface to provide a feedback signal for a droplet distribution in the vaporizer chamber. The electric current thus obtained may be measured in different locations of the vaporizer chamber wall relative the injection nozzle, which provides an efficient measure for the dispersal achieved.

The first surface may be electrically connected to an identical polarity as the injection unit and liquid for providing a repelling force and for spreading the liquid over a larger area than the first surface.

The control unit 80 may be adapted to provide a difference between the first and second potential in dependence of the specific liquid substance, such as in the range of 100V to 1000V.

The interior surface may comprise sections electrically isolated towards each other. Electrical Isolator units 61, 62, 63, 64, 65, 66, 67 of electrically non-conductive material are provided to separate the different surface sections.

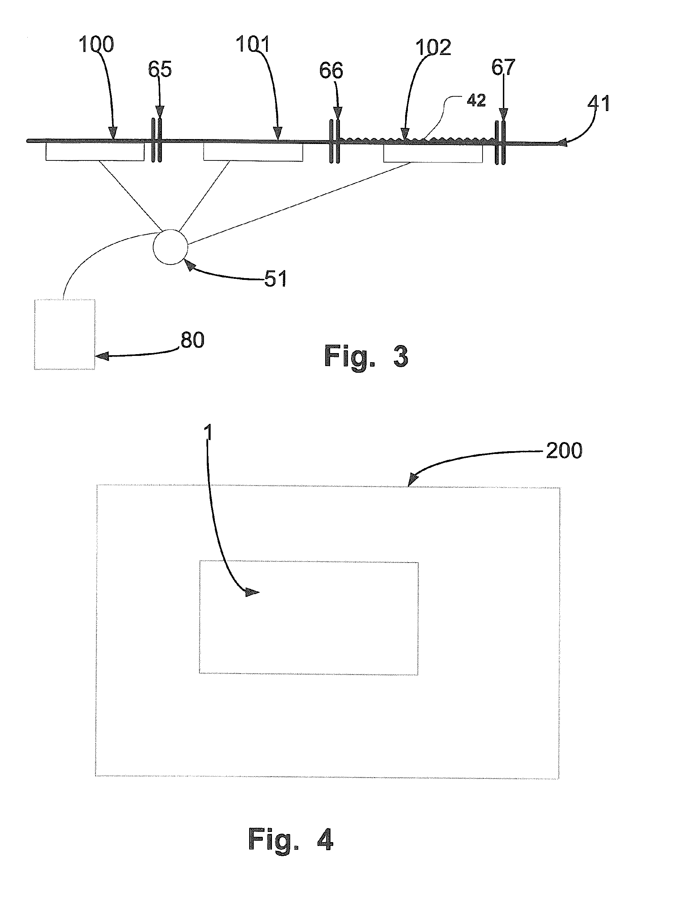

Different sections 100, 101, 102 of the interior surface are isolated from each other. They may are connected to different electrical potentials, see Fig. 3. The control unit 80 may thus be adapted to provide different potential differences between the first and second potential such that droplets of different size are directed towards different landing positions.

In this manner droplets of different size may be directed towards different landing positions. When surface portion to which larger droplets are directed, more heating power may be used at such surface portions, further increasing efficiency of the vaporization process.

The control unit 80 may be adapted to provide a time variable difference between the first and second potential in order to provide a desired pattern of attraction and repulsion of droplets of the liquid substance in the vaporizer chamber 40. The liquid may thus be supplied to different evaporation surfaces during a pulse or between different pulses, as described above. This reduced the temperature drop at each specific evaporation area. Vaporization is advantageously improved.

In addition, the injected spray may be directed onto an enlarged evaporation surface to further increase efficiency of the vaporization of the anesthetic agent liquid. The evaporation surface 41 may comprise an enlarged surface area, such as shown by the enlarged surface area 42 in Fig. 2 or 3. The enlarged surface area may comprise one or more conical portion(s) of the interior surface of the gas channel into which a spray is generated. A conical evaporation surface may in addition be enlarged by secondary enlarged surface areas. The enlarged surface area of evaporation surface 41 may for this purpose be corrugated or porous. The enlarged surface area of evaporation surface 41 allows for an increased spreading of the liquid to a larger surface area and thus further improved vaporization efficiency.

The interior surface may comprise a conical surface as an enlarged surface portion oriented towards a nozzle of the injection unit, as illustrated in Figs. 1 or 2.

Alternatively, or in addition, the dispersal unit 50 may comprise an acoustic generator unit 58 for generating at least one sound wave in the vaporizer chamber to deflect the spray therein. The acoustic generator unit 58 is preferably arranged to provide the sound wave substantially transversal to a direction of travel of the spray 32 from the injection unit 30 such that a change of direction of travel of the spray 32 is provideable by the acoustic generator unit 58.

The sound generator, such as a loudspeaker, is preferably operated at a non-audible frequency to not disturb users with audible noise. The sound generator is not immersed or submersed in the liquid substance. The sound wave does not generate a spray, it is already created by the injection unit 30 and merely acted upon by means of the sound wave. It is rather preferably arranged at a distance from the spray, e.g. in the vaporizer chamber wall. For instance trains of sound pulses may be directed towards the spray. Depending on the spatial arrangement of the acoustic generator and the direction of the sound wave, the spray is contactless acted upon. The spray may thus be actively dispersed. The sound wave is for instance sent substantially transversal to the direction of travel of the spray from nozzle. Thus, a change of direction of travel of the spray is provideable by the acoustic generator unit.

An injection nozzle 31 of the injection unit may additionally have an adjustable nozzle distribution angle to further improve a distribution angle of the initial spray before controllably being dispersed. Thus the effective vaporizing surface area may be further improved by an increased distribution angle of the spray. However, increasing the lobe of the spray has its limitations as injection gets less precise and controllable.

A temperature measurement unit 95 may arranged to measure a temperature in the vaporizer chamber 40. The control unit 80 may thus be adapted to increase a dispersal of the spray 32 based on a temperature drop in vaporizer chamber 40 that is larger than a threshold temperature difference between a temperature measured before the injection and within a time interval after the injection of the spray.

A breathing apparatus 200 comprising an injection device 1 is schematically illustrated in Fig. 4.

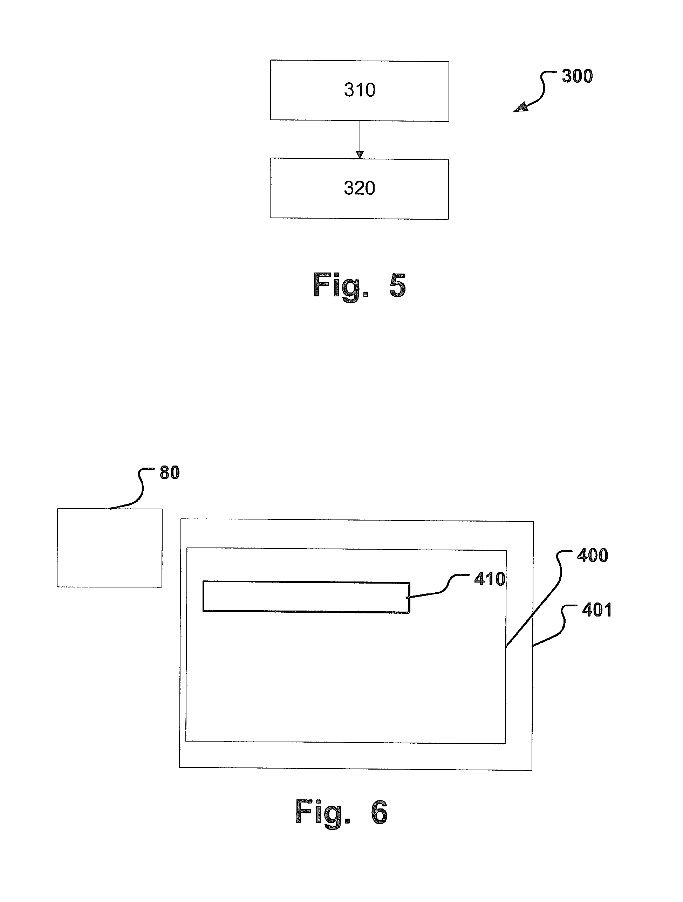

As illustrated in Fig. 5, a method 300 of internally controlling an injection device 1 for improving vaporization of a liquid substance comprises the following steps. A spray of the liquid substance is injected 310 into a vaporizer chamber 40 towards a first surface portion of an interior surface of the vaporizer chamber. The method further comprises and contactless acting on at least a portion of the spray of the liquid in the vaporizer chamber for dispersing at least the portion of the spray relative the first receiving surface portion when injected into the vaporizer chamber.

The contactless acting may be made in dependence of a temperature change in the vaporizer chamber.

A dispersal of the spray may be increased based on a temperature drop in the vaporizer chamber upon injection of the spray.

The method may comprise distributing the spray over a larger area than without the dispersal unit.

The method may comprise controlling a control a movement of droplets of the spray within the vaporizer chamber.

In Fig. 6, a computer program 400 embodied on a computer-readable medium 401 is illustrated for processing by a computer, such as the control unit 80. The computer program 400 comprises a code segment 410 for controlling a dispersal unit for increasing dispersal of the spray based on a temperature drop in the vaporizer chamber upon injection of the spray, when injecting a spray of the liquid substance into a vaporizer chamber towards a first surface portion of an interior surface of the vaporizer chamber. Dispersal is made by the dispersal unit by contactless acting on at least a portion of the spray of the liquid in the vaporizer chamber for dispersing at least the portion of the spray relative a first receiving surface portion when injected into the vaporizer chamber.

The present invention has been described above with reference to specific embodiments. However, other embodiments than the above described are equally possible within the scope of the invention. Different method steps than those described above, performing the method by hardware or software, may be provided within the scope of the invention. The different features and steps of the invention may be combined in other combinations than those described. The scope of the invention is only limited by the appended patent claims.

## Claims

1. An injection device for vaporizing a liquid substance in a breathing apparatus comprising
a vaporizer chamber having an interior surface having at least a first surface portion,
an injecting unit for generating a spray of said liquid substance into said vaporizer chamber towards said first surface portion, and
a dispersal unit for contactless acting on at least a portion of said spray of said liquid in said vaporizer chamber relative said first surface portion when injected into said vaporizer chamber.

2. The device of claim 1, wherein said dispersal unit comprises an electric charge unit for providing a first electrical potential to at least a portion of said interior surface and a second electrical potential, different from said first electrical potential, to said substance.

3. The device of claim 2, wherein said electric charge unit comprises an electrical potential source operatively in electrical contact with said liquid substance, and/or said electrical potential source is operatively in electrical contact with said spray upon injection from said injection unit.

4. The device of claim 3, wherein said electrical potential source is electrically connected with an electrically conducting portion at said injection unit, such as of a nozzle, a flow channel, and/or a container for holding or conveying said liquid, and/or an electrically conductive body, such as a net, in said flow channel, nozzle or container, arranged to be in electrical contact with said liquid prior to being injected into said vaporizer chamber; and/or
said electric charge unit comprises an electrically conductive body arranged to contact said spray of said liquid substance upon injection from said injection unit before reaching said first surface portion.

5. The device of any of claims 2 to 4, wherein said device comprises a control unit adapted to vary a difference between said first electrical potential and said second electrical potential for providing an attraction or repulsion of said spray in relation to said interior surface in selectable intensity.

6. The device of any of claims 2 to 5, wherein said device comprises a measurement unit for a potential difference signal between said first and second potential.

7. The device of claim 6, wherein said device comprises a control unit adapted to control an electric charge distribution with regard to spatial distribution and intensity in said interior surface based on said potential difference signal and a mass of droplets of said spray; and/or wherein said measurement unit is arranged to measure an electric charge received over time at said interior surface to provide a feedback signal for a droplet distribution in said vaporizer chamber.

8. The device of any of claims 2 to 7, wherein said injecting unit and said liquid are electrically connected to said first potential and at least a portion of said interior surface is connected to said second potential of said electric charge unit for locally generating an electrical attraction force and/or repulsion force in said interior surface for said spray.

9. The device of claim 8, wherein said first surface is electrically connected to an identical polarity as said injection unit and liquid for providing a repelling force and for spreading the liquid over a larger area than said first surface.

10. The device of any of claims 2 to 9, wherein said device comprises a control unit that is adapted to provide a difference between said first and second potential in dependence of said specific liquid substance; and/or wherein said interior surface comprises sections electrically isolated from each other; and/or wherein said interior surface comprises sections electrically isolated from each other, and wherein different sections of said interior surface are connected to different electrical potentials.

11. The device of any of claims 2 to 10, comprising a control unit adapted to provide a time variable difference between said first and second potential in order to provide a desired pattern of attraction and repulsion of droplets of said liquid substance in said vaporizer chamber; and/or comprising a control unit adapted to provide different potential differences between said first and second potential difference between said first electrical potential and said second electrical potential such that droplets of different size are directed towards different landing positions

12. The device of any of claims 1 to 11, wherein said dispersal unit comprises an acoustic generator unit for generating at least one sound wave in said vaporizer chamber to deflect said spray therein, wherein said acoustic generator unit is preferably arranged to provide said sound wave substantially transversal to a direction of travel of said spray from said injection unit such that a change of direction of travel of said spray is provideable by said acoustic generator unit.

13. The device of any of claims 1-12, comprising a temperature measurement unit arranged to measure a temperature in said vaporizer chamber, and a control unit adapted to increase dispersal of said spray based on a temperature drop that is larger than a threshold temperature difference between a temperature measured before said injection and within a time interval after said injection of said spray.

14. A method of internally controlling an injection device for improving vaporization of a liquid substance, said method comprising
injecting a spray of said liquid substance into a vaporizer chamber towards a first surface portion of an interior surface of said vaporizer chamber,
and contactless acting on at least a portion of said spray of said liquid in said vaporizer chamber for dispersing at least said portion of said spray relative said first receiving surface portion when injected into said vaporizer chamber.

15. The method of claim 14, wherein said contactless acting is made in dependence of a temperature change in said vaporizer chamber; or wherein said contactless acting is made in dependence of a temperature change in said vaporizer chamber, and comprising increasing a dispersal of said spray based on a temperature drop in said vaporizer chamber upon injection of said spray; and/or wherein said injection device is the device of any of claims 1-13.

16. A computer program embodied on a computer-readable medium for processing by a computer, the computer program comprising
a code segment for controlling a dispersal unit for increasing dispersal of said spray based on a temperature drop in said vaporizer chamber upon injection of said spray, when injecting a spray of said liquid substance into a vaporizer chamber towards a first surface portion of an interior surface of said vaporizer chamber, and whereby dispersal is made by said dispersal unit by contactless acting on at least a portion of said spray of said liquid in said vaporizer chamber for dispersing at least said portion of said spray relative a first receiving surface portion when injected into said vaporizer chamber.
